# EUROPEAN PATENT SPECIFICATION

(11) **EP 0 686 044 B1**
(45) Date of publication and mention of the grant of the patent: **02.09.1998**
(21) Application number: 94909627.5
(22) Date of filing: 17.02.1994
(51) Int. Cl.: A61K 39/395

(54) **INHIBITION OF INTIMAL HYPERPLASIA USING ANTIBODIES TO PDGF RECEPTORS**
VERWENDUNG VON ANTIKÖRPERN GEGEN PDGF-REZEPTOREN ZUR INHIBIERUNG VON HYPERPLASIE DER INTIMA
INHIBITION DE L'HYPERPLASIE DE L'INTIMA PAR ANTICORPS DES RECEPTEURS DU FACTEUR DE CROISSANCE DERIVE DES PLAQUETTES

(30) Priority: 25.02.1993 US 23504
(43) Date of publication of application: 13.12.1995
(73) Proprietor: ZymoGenetics, Inc., Seattle, WA 98102 (US); THE UNIVERSITY OF WASHINGTON, Seattle, WA 98195 (US)
(72) Inventor: HART, Charles E., Brier, WA 98036 (US); KENAGY, Richard D., Seattle, WA 98125 (US); CLOWES, Alexander W., Seattle, WA 98122 (US)
(74) Representative: Jorgensen, Dan
(86) International application number: US9401611
(87) International publication number: WO9419016

(56) References cited:
- WO-A-93/10805
- AMERICAN JOURNAL OF CARDIOLOGY vol. 60, no. 3 , 31 July 1987 , NEW YORK NY, USA pages 20B - 28B L. HARKER 'Role of platelets and thrombosis in mechanisms of acute occlusion and restenosis after angioplasty.'
- JOURNAL OF INVESTIGATIVE DERMATOLOGY vol. 96, no. 6 , June 1991 , BALTIMORE MD, USA pages 983 - 986 J. KRANE ET AL. 'Increased dermal expression of platelet-derived growth factor receptors in growth-activated skin wounds and psoriasis.'

## Description

### Technical Field

The present invention relates to methods for inhibiting intimal hyperplasia, including restenosis, in a mammal following vascular injury, and to compositions useful within those methods.

### Background of the Invention

Proliferation of smooth muscle cells (SMCs) in the vessel wall is an important event in the formation of vascular lesions in atherosclerosis, after vascular reconstruction or in response to other vascular injury. For example, treatment of atherosclerosis frequently includes the clearing of blocked vessels by angioplasty, endarterectomy or reduction atherectomy, or by bypass grafting, surgical procedures in which atherosclerotic plaques are compressed or removed through catheterization (angioplasty), stripped away from the arterial wall through an incision (endarterectomy) or bypassed with natural or synthetic grafts. These procedures remove the vascular endothelium, disturb the underlying intimal layer, and result in the death of medial SMCs. This injury is followed by medial SMC proliferation and migration into the intima, which characteristically occurs within the first few weeks and up to six months after injury and stops when the overlying endothelial layer is reestablished. In humans, these lesions are composed of about 20% cells and 80% extracellular matrix.

In about 30% or more of patients treated by angioplasty, endarterectomy or bypass grafts, thrombosis and/or SMC proliferation in the intima causes reocclusion of the vessel and consequent failure of the reconstructive surgery. This closure of the vessel subsequent to surgery is known as restenosis.

A similar process of SMC proliferation has also been observed in organ transplants, and may contribute to transplant atherosclerosis and organ failure. The intimal thickening in this process involves only the grafted organ.

It has been postulated that platelet mitogens, such as platelet derived growth factor (PDGF), play a role in the development of atherosclerotic plaques (see Ross et al., Cell 46: 155-169, 1986; Harker, Am. J. Cardiol. 60: 20B-28B, 1987). One proposed mechanism for plaque formation is the release by platelets, at sites of endothelial denudation, of growth factors that stimulate SMC growth (Ross and Glomset, N. Eng. J. Med. 295: 369-377, 420-425, 1976; Ross, Arteriosclerosis 1: 293-311, 1981). Moore et al. (Thrombos. Haemostas. (Stuttg.) 35: 70, 1976) and Friedman et al. (J. Clin. Invest. 60: 1191-1201, 1977), using an indwelling catheter injury model, reported an inhibition of experimentally induced intimal lesion formation in rabbit arteries by prolonged thrombocytopenia induced by administration of anti-platelet serum. It has also been postulated that SMCs may themselves produce PDGF which stimulates lesion development through an autocrine mechanism (Ross et al., ibid; Walker et al., Proc. Natl. Acad. Sci. USA 83: 7311-7315, 1986). Fingerle et al. (Proc. Natl. Acad. Sci. USA 86: 8412-8416, 1989) investigated intimal lesion formation in thrombocytopenic rats and concluded that platelets do not play a role in the initial SMC proliferation after balloon injury but may regulate SMC migration into the intima. Platelets are now known to release a number of growth factors, including PDGF, epidermal growth factor (EGF), transforming growth factors alpha and beta (TGFα and TGFβ), insulin-like growth factor I (IGF-I) and platelet derived endothelial cell growth factor, as well as several chemoattractant molecules.
Krane et al. (J. Invest. Dermatol. 96: 983-986, 1991) examined the expression pattern of PDGF receptors in cryostat sections of normal and growth-activated human skin using a monoclonal antibody specific for the beta subunit of the PDGF receptor. PDGF receptors were expressed at low levels in normal skin, with only occational staining of dermal connective tissue cells. In contrast, PDGF receptor expression was greatly elevated in the dermis of growth-activated skin from 15 chronic wounds and 10 psoriatic lesions. WO 93/10805 (COR THERAPEUTICS, Inc.) published after the present application's priority date relates to the production and use of immunoglobulin polypeptides that inhibit PDGF-mediated proliferation of cells displaying the human type beta platelet derived growth factor receptor.

Although certain studies implicate PDGF in processes associated with lesion development, no studies have shown the participation of PDGF in these processes in primates.

Removal of atherosclerotic plaques by angioplasty or endarterectomy has limited efficacy, and no effective treatment for restenosis of treated vessels or stenosis of bypass grafts has been developed. There is therefore a need in the art for a medicament for use in reducing or preventing the development of SMC-rich lesions in vascular walls, including stenosis of blood vessels following vascular injury, such as injury due to balloon catheterization, endarterectomy or reduction atherectomy, as well as in vascular grafts, organ transplants and catheter emplacements. The present invention provides such medicaments and fulfills other, related needs.

The present invention relates to the use of an anti-PDGF receptor antibody which inhibits binding of PDGF to the PDGF receptor for the preparation of a medicament for use in inhibiting intimal hyperplasia in the vasculature of a mammal, subject to the limitation that said antibody does not specifically bind to the fifth Ig-like domain of the human PDGF-beta receptor. In one preferred embodiment said mammal is a primate. In another preferred embodiment, said antibody is a monoclonal antibody. In a further preferred embodiment, said antibody is an anti-PDGF-alpha receptor antibody. In a still further preferred embodiment,said antibody is an anti-PDGF-beta receptor antibody.

The present invention also relates to the use of an anti-PDGF receptor antibody which inhibits binding of PDGF to the PDGF receptor for the preparation of a medicament for administration to a mammal prior to an acute vascular injury, for inhibiting intimal hyperplasia in the vasculature of the mammal, subject to the limitation that said antibody does not specifically bind to the fifth Ig-like domain of the human PDGF-beta receptor. In one preferred embodiment, said injury is due to vascular reconstruction. In another preferred embodiment, said vascular reconstruction comprises angioplasty, endarterectomy, atherectomy, or vascular graft emplacement. In a further preferred embodiment, said antibody is administered prior to or in combination with emplacement of a vascular graft. In a still further preferred embodiment, said antibody is administered subsequent to an acute vascular injury. In a still further preferred embodiment, said injury is due to vascular reconstruction. In a yet still further embodiment, said vascular reconstruction comprises angioplasty, endarterectormy, atherectomy or vascular graft emplacement.

The present invention also relates to the use of a panel of anti-PDGF receptor antibodies which inhibit binding of PDGF to the PDGF receptor for the preparation of a medicament for use in inhibiting intimal hyperplasia in the vasculature of a mammal, wherein said panel of anti-PDGF receptor antibodies is capable of neutralizing the AA, AB and BB isoforms of PDGF to PDGF receptors, subject to the limitation that said panel of antibodies does not specifically bind to the fifth Ig-like domain of the human PDGF-beta receptor. In one preferred embodiment, said panel of anti-PDGF receptor antibodies comprises anti-PDGF-alpha receptor antibodies and anti-PDGF-beta receptor antibodies. In another preferred embodiment, said panel of antibodies comprises a humanized monoclonal antibody. In another preferred embodiment, said antibody is a single chain antibody. In another preferred emdodiment, said antibody is a chimeric antibody. In another preferred embodiment, said antibody is a human-mouse chimeric antibody. In another preferred embodiment, said chimeric antibody comprises mouse variable domains operably linked to human constant domains. In another preferred embodiment, said intimal hyperplasia occurs within a vascular graft or transplanted organ.

The present invention also relates to the use of an anti-PDGF receptor antibody which inhibits binding of PDGF to the PDGF receptor for the preparation of a medicament for use in inhibiting smooth muscle cell proliferation at a site of vascular injury in a mammal, wherein said antibody is selected from the group consisting of anti-PDGF-alpha receptor antibodies and anti-PDGF-beta receptor antibodies, subject to the limitation that said antibody does not specifically bind to the fifth Ig-like domain of the human PDGF-beta receptor. In one preferred embodiment, said mammal is a primate.

### Brief Description of the Drawings

Figure 1 illustrates the binding of anti-PDGF receptor monoclonal antibodies to cells that express recombinant PDGF-beta receptor. Results are expressed as mean cpm bound of ¹²⁵I-rabbit anti-mouse IgG for triplicate determinations. The bars indicate standard deviation.

Figure 2 illustrates the binding of anti-PDGF receptor monoclonal antibodies to cells that express recombinant PDGF-alpha receptor. Results are expressed as mean cpm bound of ¹²⁵I-rabbit anti-mouse IgG for triplicate determinations. The bars indicate standard deviation.

Figures 3A-3C illustrate the neutralization of PDGF mitogenic activity on human dermal fibroblasts by anti-PDGF receptor monoclonal antibodies. The results are presented as the mean level of [³H]thymidine incorporation for each of the PDGF ligand test conditions. Standard deviation is shown by the T at the top of each bar. Each panel also shows a standard curve for PDGF ligand alone. A) PDGF-AA stimulation, B) PDGF-AB stimulation, C) PDGF-BB stimulation.

Figure 4 illustrates the neutralization of PDGF-AA mitogenic activity on baboon smooth muscle cells by anti-PDGF receptor monoclonal antibodies. A standard curve for ligand alone is shown on the left. Results are presented as the mean level of [³H] thymidine incorporation. Standard deviation is shown by the T at the top of each bar.

Figure 5 illustrates the neutralization of PDGF-AB mitogenic activity on baboon smooth muscle cells by anti-PDGF receptor monoclonal antibodies. A standard curve for ligand alone is shown on the left. Results are presented as in Figure 4.

Figure 6 illustrates the neutralization of PDGF-BB mitogenic activity on baboon smooth muscle cells by anti-PDGF receptor monoclonal antibodies. A standard curve for ligand alone is shown on the left. Results are presented as in Figure 4.

Figures 7 A and B illustrate titration of representative monoclonal antibodies to neutralize the mitogenic activity of PDGF-AA on baboon smooth muscle cells. The results are presented as the mean level of [³H] thymidine incorporation for each of the PDGF-AA test conditions. Standard deviation is shown by the T for the PDGF-AA standard curve samples. (A) Standard curve of PDGF-AA mitogenic activity. (B) Inhibitory potency of MAbs 169.14 and 169.31 for PDGF-AA mitogenic activity as shown by a decrease in the level of [³H] thymidine incorporation.

Figure 8 illustrates the neutralization of baboon serum mitogenic activity on baboon smooth muscle cells by anti-PDGF receptor monoclonal antibodies. A standard curve for serum alone is shown on the left. The results are presented as the mean level of [³H] thymidine incorporation. Standard deviation is shown by the T at the top of each bar.

Figure 9 illustrates the mitogenic activity of baboon serum, in the presence of monoclonal antibody 169.31, on baboon smooth muscle cells.

### Detailed Description of the Invention

As noted above, restenosis of blood vessels is a common problem in patients who have undergone angioplasty or endartarectomy. Restenosis is one example of intimal hyperplasia, which is believed to proceed via a process that includes both proliferation (mitosis) and migration of vascular smooth muscle cells in the area damaged by the surgical procedure, as well as by the production of extracellular matrix. This proliferative process is also manifested in the occlusion of vascular grafts (both natural, including autologous and allogeneic, and synthetic), and in transplanted organs. This proliferative process results in the development of lesions rich in smooth muscle cells and is refered to herein as intimal hyperplasia.

The present invention provides medicaments for use in inhibiting the development of SMC-rich lesions through the use of antibodies against PDGF receptors. Such lesions result in the partial or complete blocking of a blood vessel through intimal thickening (hyperplasia). Inhibition of intimal hyperplasia will be understood to include interfering with the proliferative process by reducing or preventing one or more of cell migration, cell mitosis, and extracellular matrix formation. By blocking mitosis and/or migration through interfering with the interaction of PDGF and its receptors, SMC proliferation and subsequent matrix deposition may be reduced.

Antibodies useful within the present invention may be produced by conventional procedures of immunization and purification. Briefly, a PDGF receptor, receptor fragment or fusion protein comprising receptor polypeptide, preferably purified, is administered to an animal such as a mouse, rat, rabbit or goat in an amount sufficient to cause an immune response. It is preferred to administer the growth factor receptor in combination with an adjuvant, such as Freund's adjuvant, in order to enhance the immune response. Although a single injection of antigen may be sufficient to induce antibody production in the animal, it is generally preferred to administer a large initial injection followed by one or more booster injections over a period of several weeks to several months. See, e.g., Hurrell, J.G.R., ed., Monoclonal Hybridoma Antibodies: Techniques and Applications, CRC Press Inc., Boca Raton, FL, 1982. Blood is then collected from the animal and clotted, and antibodies are isolated from the serum using conventional techniques such as salt precipitation, ion exchange chromatography, affinity chromatography or high performance liquid chromatography.

Within one embodiment of the invention, monoclonal antibodies are used. Monoclonal antibodies provide the advantages of ease of production and lower therapeutic doses as compared to polyclonal antisera, since only antibodies of the desired specificity are used. Methods for producing monoclonal antibodies are well known in the art and are disclosed, for example, by Kohler and Milstein (Nature 256: 495, 1975; Eur. J. Immunol. 6: 511-519, 1976). See also Hurrell, J.G.R., ed., Monoclonal Hybridoma Antibodies: Techniques and Applications, CRC Press Inc., Boca Raton, FL, 1982 and Hart, U.S. Patent No. 5,094,941. As will be appreciated by those skilled in the art, antibody fragments, such as Fab fragments, may also be used.

It is generally preferred to use antibodies that are syngeneic with the patient or that contain syngeneic constant regions. For this reason, genetically engineered antibodies will generally be used in the treatment of humans. Methods for producing recombinant human antibodies or humanized non-human (i.e. chimeric) antibodies are disclosed by Cabilly et al. (U.S. Patent No. 4,816,567), Robinson et al. (WO 87/02671) and Neumaier (WO 90/00616). Briefly, human constant region genes are joined to appropriate human or non-human variable region genes. For example, the amino acid sequences which represent the antigen binding sites (CDRs, or complimentarity-determining regions) of the parent murine monoclonal antibody are grafted at the DNA level onto human variable region framework sequences. This process is known as "humanization". Methods for this technique are known in the art and are disclosed, for example, by Jones et al. (Nature 326: 522-525, 1986), Riechmann et al. (Nature 322: 323-327, 1988) and Queen et al. (Proc. Natl. Acad. Sci. USA 86: 10029-10033, 1989).

The joined genes are then transfected into host cells, which are cultured according to conventional procedures. In the alternative, monoclonal antibody producing cells may be transfected with cloned human constant region genes, and chimeric antibody genes generated by homologous recombination. Thus it is possible to assemble monoclonal antibodies with a significant portion of the structure being human, thereby providing antibodies that are more suitable for multiple administrations to human patients.

Alternatively, a single chain antibody may be developed through the expression of a recombinant polypeptide which is generally composed of a variable light-chain sequence joined, typically via a linker polypeptide, to a variable heavy-chain sequence. Methods for producing single chain antibodies are known in the art and are disclosed, for example, by Davis et al. (BioTechnology 9: 165-169, 1991).

Within the present invention it is preferred to use neutralizing antibodies. "Neutralizing antibody" is used herein to designate an amount of an antibody sufficient to block essentially all of the biological activity of an antigen in an in vitro test system, e.g. the ability to block the interaction of one or more PDGF ligands with PDGF receptor(s). Suitable in vitro test systems include, inter alia, mitogenesis assays and receptor binding assays. For example, 25 µg/ml of a monoclonal anti-PDGF-alpha receptor MAb described herein is able to block the mitogenic activity of 10 ng/ml of PDGF-AA. As will be understood by those skilled in the art, the amount of antibody needed to neutralize a given amount of antigen will depend on such factors as antibody specificity and affinity.

Two PDGF receptor polypeptides have been described. These are termed "alpha receptor" (Kelly et al., WO 90/14425; Claesson-Welsh et al., Proc. Natl. Acad. Sci. USA 86: 4917-4921, 1989) and "beta receptor" (Claesson-Welsh et al., Mol. Cell. Biol. 8: 3476-3486, 1988; Gronwald et al., Proc. Natl. Acad. Sci. USA 85: 3435-3439, 1988). In the presence of PDGF ligand, the receptor polypeptides dimerize. Three receptor subtypes are thus possible: αα, αβ and ββ. The β receptor is specific for the B-chain of PDGF, while the α receptor binds the A-chain and the B-chain. The anti-PDGF receptor antibodies used within the present invention will preferably be a panel of antibodies capable of neutralizing all three PDGF receptor isoforms (αα, ββ and αβ). As used herein, the term "panel" denotes a combination of two or more antibodies having different specificities. The antibodies may be specific for different antigens or for different epitopes on a single antigen. Monoclonal antibodies (MAbs) are preferred.

Anti-receptor monoclonal antibodies may also be used as targeting agents for the delivery of compounds of therapeutic interest. Such compounds include, but are not limited to, toxins, cytostatic compounds, or proenzymes whose potential function can be to activate endogenous proenzymes, to activate proenzymes added from exogenous sources, or to activate enzyme cleavage sites on prodrugs. Anti-receptor antibodies can also be labeled with radionucleotides, dyes, flourescent compounds or the like for use as imaging agents. Examples of this include imaging sites of thrombosis, or sites of vascular injury where there is exposure, by example, of vascular smooth muscle cells which express cell-surface receptors.

Monoclonal antibodies can also be used to develop bifunctional antibodies where there are two independent antigenic binding on each immunoglobuln molecule. This technology is known in the art and has sites been disclosed in the literature (Thromb. Res. Suppl. X: 83, 1990). Additionally, bispecific antibodies can also be constructed from single chain antibodies. This technology is known in the art and has been disclosed, for example, by A. George (The Second Annual IBC International Conference on Antibody Engineering, Dec. 16-18, 1991, San Diego CA).

Anti-PDGF receptor antibodies may be administered by bolus injection (intravenous, intramuscular, intraperitoneal or subcutaneous) prior to surgery (generally within 24 hours before surgery) and optionally continuing after surgery at intervals of from several hours to several days over the course of one to two weeks or more. In many cases it will be preferable to administer daily doses during a hospital stay, followed by less frequent bolus injections during a period of outpatient treatment. The antibodies may also be delivered by slow-release delivery systems, including such systems incorporated into vascular grafts or stents, or by way of perfusion or double balloon catheters.

The methods of the present invention are particularly useful in the treatment of intimal hyperplasia due to acute vascular injury. Acute vascular injuries are those which occur rapidly (i.e. over days to months), in contrast to chronic vascular injuries (e.g. atherosclerosis) which develop over a lifetime. Acute vascular injuries often result from surgical procedures such as vascular reconstruction, wherein the techniques of angioplasty, endarterectomy, atherectomy, vascular graft emplacement or the like are employed. Hyperplasia may also occur as a delayed response in response to, e.g., graft emplacement or organ transplantation.

Doses of antibody will be selected on the basis of neutralization criteria as described above. Dosage levels are calculated from neutralization data after determining clearance of antibody from the blood. In general, dosage is selected with the goal of maintaining circulating levels of antibody sufficient to neutralize any released PDGF. In general, doses will be in the range of about 20 µg to 500 mg or more of antibody per kg of patient body weight per day, preferably about 0.1 mg to 20 mg/kg, more preferably about 1 mg-10 mg/kg/day. Somewhat higher doses may be required if two or more antibodies are administered in combination. Treatment may be continued for up to six months after initial injury.

An "effective amount" of an anti-PDGF receptor antibody is defined as the amount sufficient to significantly inhibit mitogenesis and/or migration of vascular smooth muscle cells. A "significant" reduction is a reduction of mitogenesis or migration of 50% or more in an in vitro assay. While the actual amount will depend in part on such factors as the specificity and binding affinity of a particular antibody, an effective amount can be determined empirically by in vitro and ex vivo procedures known in the art and disclosed herein. In general, amounts of antibody for therapeutic use will be sufficient to provide a concentration in the bloodstream or at the site of action at least equal to that shown to be effective in vitro or ex vivo. It is preferred, however, to use higher amounts in vivo, up to or exceeding an order of magnitude increase.

For use within the present invention, anti-PDGF receptor antibodies are formulated into injectable compositions according to conventional procedures and packaged in sterile containers. The antibodies may be combined with a suitable diluent such as sterile saline or sterile water. The antibody compositions may further contain carriers, stabilizers and excipients such as sugars (e.g. mannitol) or albumin. In the alternative, the antibodies may be provided in lyophilized form and reconstituted in a suitable diluent prior to use. These compositions may be packaged in single or multiple dosage form, for example in sealed ampoules or vials.

For inhibition of stenosis in vascular grafts, anti-PDGF receptor antibodies may be covalently attached to the graft through their constant regions or incorporated into the graft in slow-release formulations.

The following examples are offered by way of illustration, not by way of limitation.

### Examples

Example 1 discloses the preparation of hybridomas producing monoclonal antibodies to the PDGF receptor alpha and beta polypeptides. Examples 2, 3 and 4 disclose the identification and characterization of anti-PDGF-beta receptor monoclonal antibodies. Example 5 discloses the identification and characterization of anti-PDGF-alpha receptor monoclonal antibodies. Example 6 discloses the determination of the binding specificities of certain representative monoclonal antibodies. Example 7 demonstrates the neutralization of PDGF mitogenic activity on human dermal fibroblasts using anti-PDGF receptor monoclonal antibodies. Example 8 demonstrates the neutralization of PDGF mitogenic activity on baboon smooth muscle cells using anti-PDGF receptor monoclonal antibodies. Examples 9 and 10 disclose the use of anti-PDGF receptor monoclonal antibodies to neutralize baboon serum mitogenic activity. Example 11 demonstrates the inhibition of baboon aortic smooth muscle cell migration by anti-PDGF receptor monoclonal antibodies. Example 12 demonstrates the ability of anti-PDGF receptor MAbs to neutralize PDGF up to eight hours after the ligand has bound to receptors. Example 13 discloses the displacement of receptor-bound PDGF from human osteosarcoma cells by anti-PDGF receptor MAbs.

Recombinant PDGF AA and BB were produced in yeast essentially as disclosed in U.S. Patents Nos. 4,889,919; 4,845,075 and 5,037,743, and purified to homogeneity from concentrated cell culture media by a combination of cation exchange chromatography, reverse-phase chromatography, gel filtration and (NH₄)₂S0₄ fractionation. PDGF AB was prepared from outdated human platelets as disclosed by Hart et al., (Biochemistry 29: 166-172, 1991). PDGF-AA, AB and BB were labeled with ¹²⁵I by use of Iodobeads® (Pierce Chemical Co., Rockford, IL) as previously described (Hart et al. ibid.) A mutant form of B-chain termed BB_{tyr}, which has a tyrosine residue at position 23 of the mature coding sequence in place of phenylalanine, was used for iodination of PDGF-BB. Rabbit anti-mouse IgG was similarly radiolabeled with ¹²⁵I using Iodobeads®.

Fusion proteins comprising a human IgG heavy or light-chain joined to the extracellular domain of either the PDGF-alpha receptor or PDGF-beta receptor were prepared essentially as disclosed in U.S. Patent No. 5,155,027; U.S. Patent Application Serial No. 07/634,510 and EP 325,224. In once case mouse myeloma cells were transfected with cDNAs for both heavy-chain and light-chain/PDGF receptor extracellular domain fusion proteins. These cells secrete into their culture media a molecule which is analogous to human IgG in that it is composed of two light-chain and 2 heavy-chain fusion proteins. This compound is designated as tetrameric IgG/PDGFr. In another case a cDNA for light-chain/PDGF receptor extracellular domain fusion protein was transfected into the cells alone. These cells secrete monomeric light-chain fusion proteins into their culture media, designated as monomeric IgG/PDGFr. The alpha- and beta-receptor fusion proteins were designated IgG/PDGFr-alpha (tetrameric) and IgG/PDGFr-beta (monomeric and tetrameric), respectively. The fusion proteins were purified by either immunoaffinity purification using anti-PDGF receptor monoclonal antibodies, or by protein A-sepharose chromatography.

### Example 1

### Preparation of PDGF Receptor Monoclonal Antibodies

Fusion proteins comprising an IgG constant region joined to the extracellular domain of either the PDGF-alpha receptor or the PDGF-beta receptor were prepared essentially as disclosed in U.S. Patent No. 5,155,027. The alpha and beta receptor fusions were designated IgG/PDGFr-alpha and IgG/PDGFr-beta, respectively. The monomeric IgG/PDGFr-beta was expressed as a fusion of a human kappa light chain constant region and the PDGF-beta receptor extracellular domain. The tetrameric IgG/PDGFr-beta was prepared by coexpression of the monomeric construct with a human Ig heavy chain constant region plus hinge sequence fused to the extracellular domain. Alpha receptor fusions were prepared by similar means.

Eight-week-old Balb/c mice were immunized with either purified monomeric or tetrameric IgG/PDGFr-beta or purified tetrameric IgG/PDGFr-alpha. Mice were given intraperitoneal (ip) injections of approximately 10 µg of purified IgG/PDGFr mixed with complete Freund's adjuvant. At approximately 2 week intervals the mice received additional ip injections of IgG/PDGFr-beta or IgG/PDGFr-alpha mixed with incomplete Freund's adjuvant.

Hybridomas were prepared from the immunized mice essentially as disclosed in U.S. Patent No. 5,094,941. Briefly, spleen cells were isolated from the mice and washed. Contaminating red blood cells were removed by lysing with distilled water, and the spleen cells were washed. Any remaining contaminating tissue material was removed by centrifugation.

The NS-1 mouse myeloma cell line (ATCC TIB 18) was used for the fusion. To optimize fusion efficiency, cells were assayed for fusion efficiency, and a clone with a high fusion efficiency was selected. The NS-1 cells were grown in NS-1 media (Table I) at 37°C, 7% CO₂.

Thymocytes obtained from baby mice were used as a feeder layer to condition the culture medium for the cell fusions. Thymus glands were obtained from three- to four-week old Balb/c mice, and thymocytes were isolated as disclosed in U.S. Patent No. 5,094,941.

NS-1 cells were added to the prepared immunized mouse spleen cells and fusion was carried out essentially as disclosed in U.S. Patent No. 5,094,941. The cells were cultured in NS-1 medium containing 1 x HAT (Table I) and 2.5 x 10⁶ thymocytes per ml. The hybridomas were tested between days 9 and 14 for the production of specific antibodies.

**TABLE I**

| NS-1 Medium For a 500 ml solution: | |
|---|---|
| 5 ml | 10 mM MEM non-essential amino acids (GIBCO BRL, Gaithersburg, MD) |
| 5 ml | 100 mM sodium pyruvate (Irvine, Santa Ana, CA) |
| 5 ml | 200 mM L-glutamine (GIBCO BRL) |
| 5 ml | 100x Penicillin/Streptomycin/Neomycin (GIBCO BRL) |
| 75 ml | inactivated fetal calf serum (BioCell, Carson, CA) |
| 1 gm | NaHC0₃ |
| Add RPMI 1640 (GIBCO BRL) to a total volume of 500 ml. Sterilize by filtration through a 0.22 lm filter. | |

### 100x HT Stock

- 38.5 mg: thymidine
- 136.10 mg: hypoxanthine
Dissolve the thymidine and hypoxanthine in distilled H₂O and bring volume up to 100 ml. Warm the solution to 60-70°C to dissolve the solids. After the solids have dissolved, readjust the volume to 100 ml. Sterilize by filtration through an 0.22 µm filter. Store frozen at - 20°C.

### 1000x A Stock

- 17.6 ng: aminopterin
Add sterile distilled water to the aminopterin and bring the volume to 50 ml. Add 1 N NaOH drop-wise until the aminopterin dissolves. Bring the final volume to 100 ml with distilled H₂0. Sterilize by filtration through an 0.22 lm filter. Store frozen at -20°C.

### 50x HAT

- 50 ml: 100x HT
- 5 ml: 1000x A stock
- 45 ml: distilled H₂0
Sterilize the solution by filtration through an 0.22 lm filter. Store frozen at -20°C.

### ELISA A Buffer

- 0.1 M: Na₂HCO₃, pH 9.6
- 0.02%: NaN₃

### ELISA B Buffer

This buffer may be made with 1% or 2% bovine serum albumin (BSA, Sigma, St. Louis, MO)
- 5 or 10 lg: BSA (for 1% or 2% BSA, respectively)
- 250 ll: Tween® 20 (Sigma)
- 100 mg: NaN₃
Add phosphate-buffered saline pH 7.2 (PBS, Sigma) to a final volume of 500 ml. Alternatively, the buffer may be made up as 1% or 2% BSA in ELISA Buffer C.

### ELISA C Buffer

- 500 µl: Tween® 20 (Sigma)
- 200 mg: NaN₃
Add PBS to a final volume of 1 liter.

### Reaction Buffer

- 10 ml: 0.1 M Na-Citrate, pH 5.0
- 5 mg: o-phenylenediamine Dihydrochloride (Sigma)
- 5 µl: H₂O₂ (Sigma)

### Extraction Buffer

- 100 ml: PBS
- 1.0 ml: NP-40 Detergent (1% final concentration)

### Binding Media

- 500 ml: Ham's F-12 (GIBCO BRL)
- 12 ml: 1 M Hepes pH 7.4
- 5 ml: 100x Penicillin/Streptomycin/Neomycin (GIBCO BRL)
- 1 gm: rabbit serum albumin (Sigma)

### Mito Media

For a 500 ml solution:
- 250 ml: DMEM (GIBCO BRL)
- 250 ml: Ham's F-12 (GIBCO BRL)
- 0.25 ml: 10 mg/ml stock of insulin (GIBCO BRL) to give a final concentration of 5 µg/ml
- 1 ml: 10 mg/ml stock of transferrin (Collaborative Research, Bedford, MA) to give a final concentration of 20 µg/ml
- 2 ml: 4 µg/ml stock of selenium (Aldrich Chemical, Milwaukee, WI) to give a final concentration of 5 nM
- 5 ml: 10% stock solution of bovine serum albumin (GIBCO BRL) to give a final concentration of 0.1%.

### Example 2

### Identification and Characterization of Antibodies to the PDGF Beta Receptor

Hybridomas from cell fusion 162 were tested for the production of antibodies to the PDGF-beta receptor. Assays used for identification of positive hybridomas included enzyme linked immunosorbent assays (ELISA), neutralization of ¹²⁵I-PDGF-BB binding to IgG/PDGFr-beta, and neutralization of ¹²⁵I-PDGF-BB binding to human dermal fibroblasts.

The ELISA assays were carried out in 96-well microtiter plates which had been coated with monomeric IgG/PDGFr-beta. To coat the wells, IgG/PDGFr-beta was diluted to 200 ng/ml in ELISA A buffer (Table I) and 100 µl was added to each well. The plates were incubated at 37°C for 2 hours. After incubation, the plates were washed with ELISA C buffer (Table I). The plates were then incubated with 150 µl/well of ELISA B buffer (Table I) at 37°C to block nonspecific binding sites. The buffer was removed and the wells washed with ELISA C buffer.

The test hybridoma supernatants were pooled in groups of two, and 100 µl of the pooled samples was added to each of the microtiter wells. The plates were incubated for 1 hour at 37°C. The plates were washed with ELISA C buffer, then incubated for 1.5 hours at 37°C with biotin-conjugated rabbit anti-mouse IgG (Vector Labs, Burlingame, CA). The wells were washed with ELISA C buffer, then incubated for 30 minutes at 37°C with 100 µl/well of strepavidin-horseradish peroxidase (Amersham International, Amersham, U.K.). The wells were washed again with ELISA C buffer, then incubated with reaction buffer (Table I). The reaction was stopped by the addition of 1 N H₂SO₄, and the plates were read in a Dynatech® ELISA plate reader (Dynatech Laboratories, Inc. Alexandria, VA) using a filter to monitor absorbance at 490 nm. Those wells with A₄₉₀ readings greater than 0.2 were taken as positives. The positive candidates were re-assayed by ELISA as described above to determine the individual culture wells that contained the hybridoma cells producing antibody to IgG/PDGFr-beta.

Hybridomas from cell fusion 162 were also screened for neutralization of ¹²⁵I-PDGF-BB binding to IgG/PDGFr-beta. Goat anti-human IgG (Cappel Labs, Malvern, PA) was diluted with ELISA A buffer to a final concentration of 2 µg/ml. This mixture was then added to 96-well microtiter plates, 100 µl/well, and the plates were incubated for 1.5 hours at 37°C. The wells were washed with ELISA C buffer, then incubated with 200 µl per well of ELISA B buffer to block nonspecific binding sites. The plates were washed with ELISA C buffer, then incubated for 1.5 hours with tetrameric IgG/PDGFr-beta, diluted in ELISA B buffer to a final concentration of 25 ng/ml. The wells were washed with ELISA C buffer to remove unbound IgG/PDGFr-beta.

Hybridoma supernatants were pooled in groups of two, and 100 µl of the pooled samples was added to each of the microtiter wells. The wells were incubated for 1 hour at 37°C. To each well was then added 50 µl of ¹²⁵I-PDGF-BB (approximately 50,000 cpm per well). After a 1 hour incubation at 37°C the wells were washed three times with binding media (Table I). 100 µl of 0.1M NaCitrate, pH 2.5, was added to the wells for 5 minutes at room temperature, the solution was harvested and transfered to 12x75 mm tubes, and the tubes counted in a gamma counter to determine the level of ¹²⁵I-PDGF-BB binding. Antibodies which bound to IgG/PDGFr-beta and blocked ¹²⁵I-PDGF-BB binding were detected by a decrease in the level of ¹²⁵I-PDGF-BB bound, as compared to culture media alone.

Pools of media that were determined to be positive for IgG/PDGFr-beta neutralizing antibody were rescreened using an assay format similar to that described above to identify the individual wells which contained hybridomas producing the neutralizing antibody.

Culture wells that were positive either by ELISA or by neutralization of ¹²⁵I-PDGF-BB binding were subsequently assayed in a down-regulation assay format (Hart et al., J. Biol. Chem. 262: 10780-10785, 1987) for the ability to recognize PDGF-beta receptor on human dermal fibroblasts. The binding of PDGF-BB to the PDGF-beta receptor at 37°C leads to the internalization of the receptors from the cell surface and a subsequent decrease in the number of cell-surface receptors, a phenomenon refered to as down-regulation. The fibroblasts were plated into 96-well culture dishes at 10,000 cells per well and maintained in culture media for 1-2 days prior to use. To one set of wells was added PDGF-BB at a final concentration of 100 ng/ml on the cells. The cells were incubated for 1.5 hours at 37°C. The culture media was removed from the cells, and the cells washed with phosphate buffered saline (PBS). Test culture media from the hybridoma cells was then added to duplicate wells of cells that had either received the PDGF-BB treatment or cells that had been left untreated. The cells were subsequently incubated for 2 hours at 4°C, then washed with PBS. 100 µl/well of ¹²⁵I-rabbit anti-mouse IgG (100,000 cpm/well) was added to the wells, and the cells were incubated for an additional 1.5 hours at 4°C. The cells were washed with PBS, then incubated for 5 minutes at room temperature with 100 µl/well of extraction buffer (Table I). The extracts were harvested, transfered to 12x75 mm tubes and counted in a gamma counter to determine the level of ¹²⁵I-rabbit anti-mouse IgG binding. If there is antibody in the hybridoma culture supernatants capable of recognizing cell-surface PDGF-beta receptor, then there would be a decrease in the level of ¹²⁵I-rabbit anti-mouse IgG binding to those cells that were treated with PDGF-BB to down-regulate the receptors.

Several hybridomas were identified from fusion 162 as making antibody to the PDGF-beta receptor. The hybridomas identified were twice cloned by limiting dilution to obtain individual clones making monoclonal antibody. The clones were screened for antibody production by the assays described above. One hybridoma, named 162.62, was selected for further characterization.

### Example 3

### Identification and Characterization of Hybridomas Producing Anti-PDGF Beta Receptor Antibodies

Hybridomas from cell fusion 163 were tested for the production of antibodies to the PDGF-beta receptor by a combination ELISA/PDGF binding competition assay. These assays were carried out in 96-well microtiter plates. The plates were initially coated with goat anti-human IgG, 2 µg/ml in ELISA A buffer, for 2 hours at 37°C. The plates were washed with ELISA C buffer, then incubated for 1 1/2 hours at 37°C with ELISA B buffer to block nonspecific binding sites. The plates were washed with ELISA C buffer, then either used immediately or left for 1-4 days at 4°C until use. At the time of the assay the plates were washed once with ELISA C buffer, then incubated for 1 1/2 hours at 37°C with tetrameric IgG/PDGFr-beta diluted to 25 ng/ml in binding medium. The plates were then washed with ELISA C buffer to remove unbound IgG/PDGFr-beta.

Hybridoma supernatants were pooled in groups of two wells, and 100 µl of the pooled samples was added to each of the microtiter wells. The plates were incubated for 1 hour at 37°C, then washed with binding medium. To the wells was added horseradish peroxidase-conjugated goat anti-mouse IgG (Tago, Burlingame, CA) diluted 1:1000 with binding medium. The wells were incubated for 1 hour at 37°C, then washed with binding medium to remove unbound HRP-conjugated goat anti-mouse IgG. ¹²⁵I-PDGF-BB, aproximately 26,000 cpm/well, was then added to the wells for an additional 1 hour at 37°C. The wells were washed with binding medium, then incubated with reaction buffer for development of the ELISA. The reaction was stopped by the addition of 100 µl/well of 1 N H₂SO₄ and the plates read in a Dynatech® ELISA plate reader using a filter to monitor the absorbance at 490 nm.

The contents of the wells were then transfered to 12x75 mm test tubes, and the samples were counted in a gamma counter to measure the level of ¹²⁵I-PDGF-BB binding.

The above-described assay identified hybridoma cultures producing antibody to IgG/PDGFr-beta by ELISA, as well as by the ability to block the binding of ¹²⁵I-PDGF-BB to tetrameric IgG/PDGFr-beta. Those pooled samples that were positive were subsequently reassayed using the same protocol as described above to determine the individual culture wells that contained the hybridoma cells producing antibody to IgG/PDGFr-beta.

Individual wells found to be positive for binding to IgG/PDGFr-beta were subseqently assayed for the ability to neutralize ¹²⁵I-PDGF-BB binding to human dermal fibroblasts. Human dermal fibroblasts were plated into 24-well culture dishes at approximately 20,000 cells per well. The culture media was removed from the cells, and hybridoma test culture media, 0.5 ml per well, was added to duplicate wells. As a negative control, NS-1 medium alone as added to one set of wells. To a second set of wells was added PDGF-BB at a final concentration of 20 ng/ml in NS-1 medium to determine non-specific binding of ¹²⁵I-PDGF-BB. The cells were incubated for 1 hour at 4°C, then ¹²⁵I-PDGF-BB, 100 µl/well (approximately 26,000 cpm), was added to each well. The cells were incubated for an additional 1 hour at 4°C, washed with PBS, then incubated with extraction buffer. The extracts were harvested to 12x75 mm tubes and counted in a gamma counter. Test samples that caused a decrease in ¹²⁵I-PDGF-BB binding as compared to the NS-1 medium sample were assayed as positive for the ability to inhibit PDGF-BB binding to native PDGF-beta receptor on monolayers of human dermal fibroblasts.

Several hybridomas were identified from fusion 163 to be making antibody to the PDGF-beta receptor. The hybridomas identified were twice cloned by limiting dilution to obtain individual clones making monoclonal antibody. The clones were screened for antibody production by the assays described above. One hybridoma, named 163.31, was selected for further characterization.

### Example 4

### Characterizaton of Anti-PDGF Beta Receptor MAbs 162.62 and 163.31.

MAbs 162.62 and 163.31 were compared for the ability to block the binding of ¹²⁵I-PDGF-BB to either tetrameric IgG/PDGFr-beta or to PDGF-beta receptor on human dermal fibroblasts. Neutralization of ¹²⁵I-PDGF-BB binding to IgG/PDGFr-beta was done essentially as described above for the intital screening of fusion 163. Instead of adding conditioned culture media, known amounts of antibody diluted in NS-1 medium were added simultaneously with ¹²⁵I-PDGF-BB to the IgG/PDGFr-beta coated wells. NS-1 medium alone was used as a negative control. The addition of PDGF-BB, 500 ng/ml, to NS-1 medium was used to determine the level of nonspecific binding by ¹²⁵I-PDGF-BB. The wells were incubated at 4°C for 2 1/2 hours, then washed with PBS. 100 µl of 0.1M citrate pH 2.5 was added to each well to remove the bound ¹²⁵I-PDGF-BB, the samples were transferred to 12x75 mm tubes, and the tubes were then counted in a gamma counter.

To assay binding to human dermal fibroblasts, the fibroblasts were plated at approximately 20,000 cells/well in 24-well culture dishes. The cells were used for assay 2-7 days after plating. The antibodies were diluted in binding media to the concentrations shown in Table II, then mixed with ¹²⁵I-PDGF-BB, and 0.5 ml aliquots were added to duplicate wells of fibroblasts. Binding media alone was used as the negative control, and the addition of 500 ng/ml of PDGF-BB was used to determine nonspecific binding for ¹²⁵I-PDGF-BB. The cells were incubated for 2 1/2 hours at 4°C, then washed with binding media to remove unbound ligand. The cells were then incubated with extraction buffer, and the extracts were harvested and counted in a gamma counter.

The results of the binding studies are shown in Table II. The data are presented as specific cpm bound for ¹²⁵I-PDGF-BB. Nonspecific binding, determined by the addition of 500 mg/ml of unlabeled PDGF-BB, was 260 cpm for the IgG/PDGFr-beta wells and 105 cpm for the human dermal fibroblasts, and has been subtracted from the data presented. %CB = Percent control binding.

**Table II**

| MAb Neutralization of ¹²⁵I-PDGF-BB Binding to IgG/PDGFr-beta and to Human Dermal Fibroblasts | | | | | |
|---|---|---|---|---|---|
| Mab | Conc. (µg/ml) | IgG/PDGFr | | Fibroblasts | |
| | | CPM | % CB | CPM | % CB |
| 162.62 | 1.25 | 3 | 0 | 52 | 16 |
| | 0.62 | 40 | 1 | 71 | 22 |
| | 0.31 | 71 | 2 | 96 | 30 |
| | 0.15 | 91 | 3 | 69 | 21 |
| 163.31 | 1.25 | 274 | 9 | 244 | 76 |
| | 0.62 | 499 | 16 | 372 | 116 |
| Control | | 3062 | 100 | 322 | 100 |

These results demonstrate that both MAbs 162.62 and 163.31 are potent inhibitors of PDGF-BB binding to IgG/PDGFr-beta. In contrast, MAb 162.62 is a more potent inhibitor than MAb 163.31 for PDGF-BB binding to human dermal fibroblasts

MAb 162.62 was also analyzed for the ability to displace ¹²⁵I-PDGF bound to receptors on monolayers of human dermal fibroblasts. ¹²⁵I-PDGF-BB was first incubated with monolayers of human dermal fibroblasts in 24-well culture plates. The cells were washed with PBS, then subsequently incubated for 1 hour at 4°C with either MAb 162.62, 5 µg/ml, or binding medium alone. The cells were washed, incubated with extraction buffer, and the extracts were counted in a gamma counter to determine the level of ¹²⁵I-PDGF-BB binding. To determine nonspecific binding, 500 ng/ml of unlabeled PDGF-BB was added during the first incubation step. The results, presented in Table III, show that the addition of MAb 162.62 led to a 47% displacement of prebound ¹²⁵I-PDGF-BB. Thus, MAb 162.62 is able to displace receptor-bound PDGF-BB from the surface of human dermal fibroblasts.

**Table III**

| Ability of MAb 162.62 to Displace Receptor-bound ¹²⁵I-PDGF-BB From Human Dermal Fibroblasts. | | | |
|---|---|---|---|
| 1st Inc. | 2nd Inc. | CPM Bound | BB Removal |
| ¹²⁵I-BB | Binding Media | 581 | |
| ¹²⁵I-BB | MAb 162.62 | 308 | 47 % |

The subclass for MAbs 162.62 and 163.31 were determined by ELISA using IgG/PDGFr-beta coated wells and subclass specific secondary antibody. MAb 162.62 was found to be an IgG2b isotype while MAb 163.31 was found to be an IgG1 isotype.

### Example 5

### Identification and Characterization of Hybridomas Producing Anti-PDGF Alpha Receptor Antibodies

Hybridomas from cell fusion 169 were tested for the production of antibodies to the PDGF-alpha receptor by a combination ELISA/PDGF binding competition assay. These assays were carried out in 96-well microtiter plates. The plates were initially coated with goat anti-human IgG, 2 µg/ml in ELISA A buffer, overnight at 4°C. The plates were washed with ELISA C buffer, then incubated with ELISA B buffer to block nonspecific binding sites. The plates were washed with ELISA C buffer, then incubated overnight at 4°C with tetrameric IgG/PDGFr-alpha diluted to 25 ng/ml in binding medium. The plates were then washed with ELISA C buffer to remove unbound IgG/PDGFr.

Hybridoma supernatants were pooled in groups of two, and 75 µl of the pooled samples was added to each of the microtiter wells. The plates were incubated for 1 hour at 37°C, then washed with ELISA C buffer. To the wells was added horseradish peroxidase-conjugated goat anti-mouse IgG (Tago) diluted 1:1000 with binding medium. The wells were incubated for 1 hour at 37°C, then washed with ELISA C buffer to remove unbound antibody. ¹²⁵I-PDGF-AA, approximately 25,000 cpm/well, was then added to the wells for an additional 1 hour at 37°C. The wells were washed with binding medium, then inubated with reaction buffer for development of the ELISA. The reaction was stopped by the addition of 100 µl/well of 1 N H₂SO₄ and the plates read in a Dynatech® ELISA plate reader using a filter to monitor the absorbance at 490 nm.

The contents of the wells were then transfered to 12x75 mm test tubes, and the samples were counted in a gamma counter to measure the level of ¹²⁵I-PDGF-AA binding.

This assay identified hybridoma cultures producing antibody to IgG/PDGFr-alpha by ELISA and monitored for antibody which was able to block the binding of ¹²⁵I-PDGF-AA to the tetrameric IgG/PDGFr-alpha. Those pooled samples which were positive in the initial assay were reassayed using the same protocol as described above to determine the individual culture wells that contained the hybridoma cells producing antibody for IgG/PDGFr-alpha. Several wells were identified for the presence of antibody directed against IgG/PDGFr-alpha. Of these, two were selected for further analysis, 169.14 and 169.31. Hybridomas from these wells were cloned twice by limiting dilution to obtain single clones producing monoclonal antibody against the PDGF-alpha receptor. The clones were screened using the combination ELISA/¹²⁵I-PDGF-AA binding competition assay essentially as described above.

To verify that MAbs 169.14 and 169.31 recognize native PDGF-alpha receptor on monolayers of mammalian cells, the two antibodies were analyzed for the ability to block ¹²⁵I-PDGF-AA binding to alpha T-7 cells. These cells are canine kidney epithelial cells that do not naturally express PDGF-alpha receptor, but have been transfected with a cDNA coding for the full length PDGF-alpha receptor PCT Publication WO 90/14425). These cells express approximately 100,000 recombinant receptors per cell. The alpha T-7 cells were cultured in 96-well plates to approximately 95% confluency. The culture medium was removed, and dilutions of MAbs 169.14 and 169.31 were added to the cells. Controls were NS-1 medium, and NS-1 medium containing 500 ng/ml of PDGF-BB to determine the nonspecific binding component for ¹²⁵I-PDGF-AA. To each well was added 100 µl of the test sample plus 10 µl of ¹²⁵I-PDGF-AA (approximately 22,000 cpm per well). The cells were incubated with the samples for 2 hours at 4°C, washed with PBS, then extracted with 100 µl/well of extraction buffer. The extracts were harvested and counted in a gamma counter. The results are shown in Table IV. These results demonstrate that these two MAbs recognize membrane-bound PDGF-alpha receptor in mammalian cells in addition to IgG/PDGFr-alpha.

**Table IV**

| Competition for PDGF-alpha Receptor Binding on Alpha T-7 Cells Between Anti-PDGF Alpha Receptor MAbs and ¹²⁵I-PDGF-AA | | | | |
|---|---|---|---|---|
| MAb Conc. (µg/ml) | MAb 169.14 | | MAb 169.31 | |
| | CPM | % CB | CPM | % CB |
| 1.5 | 2 | 99 | 8 | 98 |
| 0.75 | 2 | 99 | 15 | 96 |
| 0.37 | 7 | 98 | 18 | 95 |
| 0.18 | 5 | 98 | 15 | 96 |
| CPM= counts per minute of ¹²⁵I-PDGF-AA bound in the presence of antibody. The nonspecific value, 28 cpm, which was determined by the addition of 500 ng/ml of PDGF-BB to the wells, has been subtracted from the CPM values given. Control binding was 392 cpm. % CB= percent control binding. | | | | |

The subclass for MAbs 169.14 and 169.31 was determined by ELISA using IgG/PDGFr-alpha coated wells and subclass specific secondary antibody. Both MAbs 169.14 and 169.31 were assayed positive for IgG2a isotype.

### Example 6

### Binding Specificity of Anti-PDGF Receptor MAbs.

To demonstrate PDGF-receptor subunit binding specificity, MAbs 162.62, 163.31, 169.14 and 169.31 were analyzed for binding to Clone 8 cells and Alpha 1-10 cells. Clone 8 cells are BHK 570 (ATCC CRL 10314) cells that have been transfected with a gene coding for the full length human PDGF-beta receptor (Gronwald et al., Proc. Natl. Acad. Sci. USA 85: 3435-3439, 1988). These cells express approximately 500,000 human PDGF-beta receptors per cell. The Alpha 1-10 cells are BHK 570 cells that have been transfected with a cDNA coding for the full length human PDGF-alpha receptor PCT Publication WO 90/14425). These cells express approximately 1,000,000 human PDGF-alpha receptors per cell. To demonstrate binding specificity for either the PDGF-alpha or beta receptor, cell surface binding studies using the anti-PDGF receptor MAbs were done with these two cell lines.

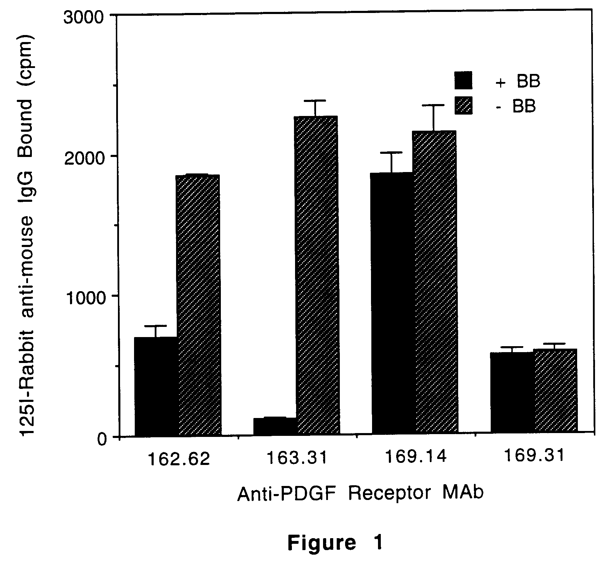

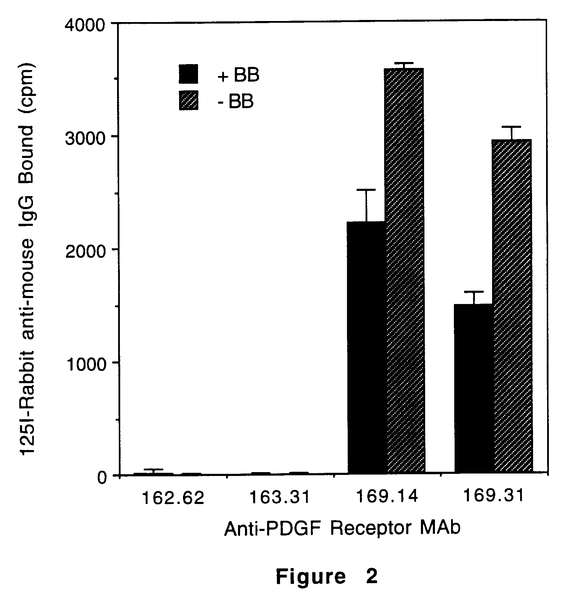

Both the Clone 8 and Alpha 1-10 cells were cultured in 24-well plates to confluency. PDGF-BB (200 ng/ml) was added to one-half of the cells to stimulate PDGF receptor down-regulation, and vehicle control (10 mm acetic acid, 0.25% rabbit serum albumin) was added to the other half. The cells were incubated for 1-2 hours at 37°C, then washed with PBS chilled to 4°C. Purified MAbs 162.62, 163.31, 169.14 and 169.31, diluted to 5 µg/ml in binding medium, were added to triplicate wells of the PDGF-BB-treated and nontreated control cells. The cells were incubated for approximately 2 hours on ice, then washed with chilled PBS to remove unbound antibody. The test wells were then incubated on ice for 30 minutes with ¹²⁵I-labeled rabbit anti-mouse IgG, diluted in binding medium to approximately 400,000 cpm/well. The wells were washed with PBS, then incubated with extraction buffer. The extracts were harvested and counted in a gamma counter. The results, shown in Figure 1, demonstrated that only MAbs 162.62 and 163.31 bound specifically to the PDGF-beta receptor, as demonstrated by the significant decrease in binding to the PDGF-BB treated Clone 8 cells when compared to untreated controls. The high level of binding by MAb 169.14 was due to elevated level of nonspecific binding by this antibody, because there was no significant decrease in ¹²⁵I-rabbit anti-mouse IgG binding to the PDGF-BB treated cells. In contrast, only MAbs 169.14 and 169.31 showed binding to the PDGF-alpha receptor as demonstrated by the specific binding to the Alpha 1-10 cells (Figure 2). Due to the ability of the antibodies to bind to cell surface PDGF receptor, these results confirm that these antibodies recogonize extracellular epitopes on the PDGF receptors.

### Example 7

### Neutralization of PDGF Mitogenic Activity on Human Dermal Fibroblasts

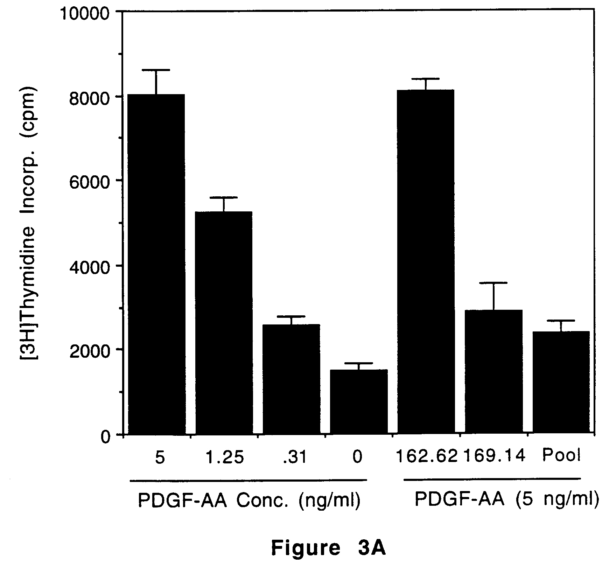

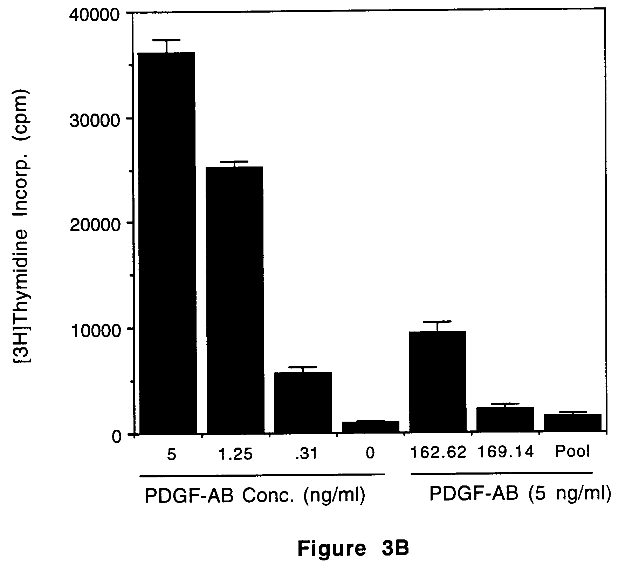

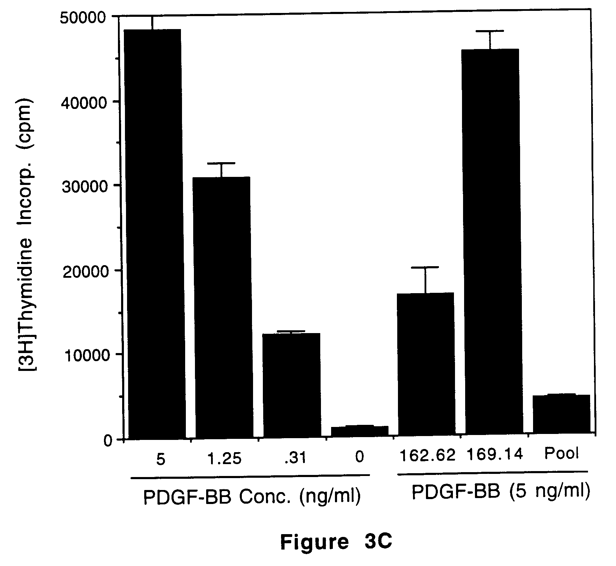

Human dermal fibroblasts were plated at approximately 20,000 cells per well in 24-well culture dishes and grown until quiescent in DMEM (GIBCO BRL) containing 2% fetal calf serum. The cells were stimulated with either PDGF-AA, AB or BB. Standard curves were run with concentrations of 5, 1.25, 0.31 and 0 ng/ml final concentration on the cells. Stock PDGF dilutions were made with 10 mM acetic acid containing 0.25% rabbit serum albumin, and 50 µl of the stock samples, or vehicle alone, was added to the culture wells to give the desired final concentrations. To analyze the ability of MAbs 162.62 and 169.14 to neutralize the mitogenic activity of each of the three PDGF ligands, 5 ng/ml of PDGF was added to wells along with 20 µg/ml (final concentration on the cells) of MAbs 162.62 and 169.14 alone, or 20 µg/ml of a pool of the two antibodies. The cells were incubated with the test samples for approximately 20 hours at 37°C. The media was aspirated, then replaced with 1 ml of DMEM containing 5% fetal calf serum and supplemented with 1 µCi/ml of [³H]thymidine. The cells were incubated for 4 hours at 37°C, washed with PBS, then harvested with trypsin and counted for [³H]thymidine incorporation in a Wallac (Turku, Finland) Betaplate® beta counter. The results, presented in Figure 3A, demonstrate that PDGF-AA mitogenic activity was neutralized by MAb 169.14 as well as by the pool, but not by MAb 162.62. PDGF-AB mitogenic activity was inhibited approximately 80% by MAb 162.62, and greater than 92% by MAb 169.14 or the antibody pool (Figure 3B). In contrast, PDGF-BB was only minimally inhibited by MAb 169.14, but was inhibited approximately 80% by MAb 162.62 and greater than 92% by the antibody pool (Figure 3C).

These results are consistent with the model of PDGF ligand binding which describes that PDGF-AA binds to PDGF-alpha/alpha receptor dimers, PDGF-AB binds to PDGF-alpha/alpha and -alpha/beta receptor dimers and PDGF-BB binds to all three PDGF receptor dimers; -alpha/alpha, - alpha/beta and -beta/beta (reviewed in Hart et al., J. Invest. Derm. 94: 535-575, 1990). Thus, if MAb 169.14 binds to and neutralizes PDGF binding to the alpha receptor, then it would be expected to neutralize essentially 100% of PDGF-AA and AB mitogenic activity, since alpha receptor binding is required for both of these ligands. This model is consistent with the results described above. The binding to and the neutralization of the PDGF-beta receptor by MAb 162.62 would then be expected to limit the amount of PDGF-AB and BB mitogenic to a level that is consistent with PDGF-AA, since AB and BB would only be able to bind to alpha/alpha dimers. Again, this is consistent with the findings of the study descibed above.

In summary, anti-PDGF-receptor MAbs 162.62 and 169.14 are able to inhibit the mitogenic activity of the three forms of PDGF in manners that are consistent with the current hypothesis as to PDGF receptor binding by the three PDGF ligands. Additionally, the use of the two antibodies in conjunction is able to inhibit essentially 100% of the PDGF mitogenic activity on human dermal fibroblasts.

### Example 8

### Neutralization of PDGF Mitogenic Activity on Baboon Smooth Muscle Cells

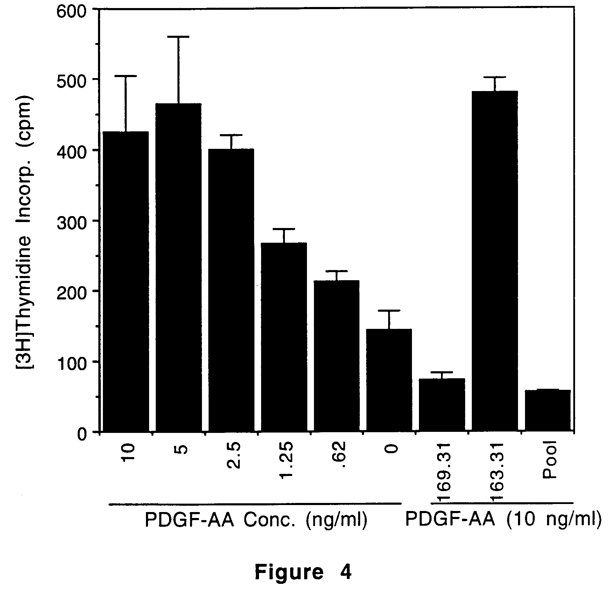

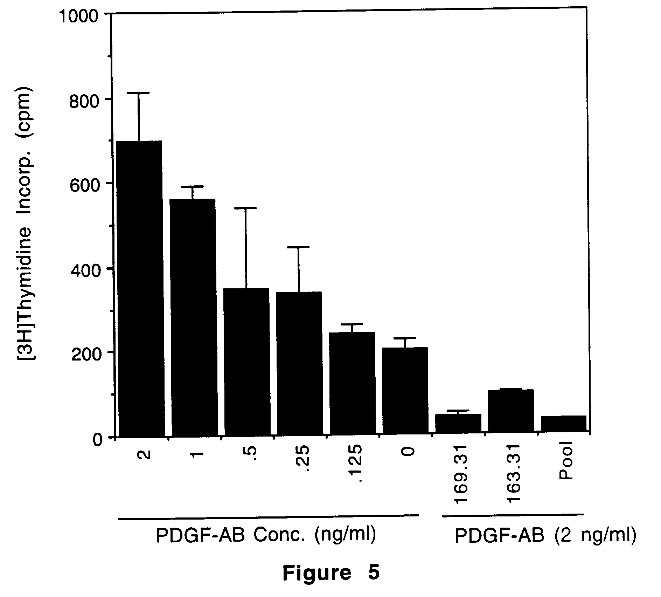

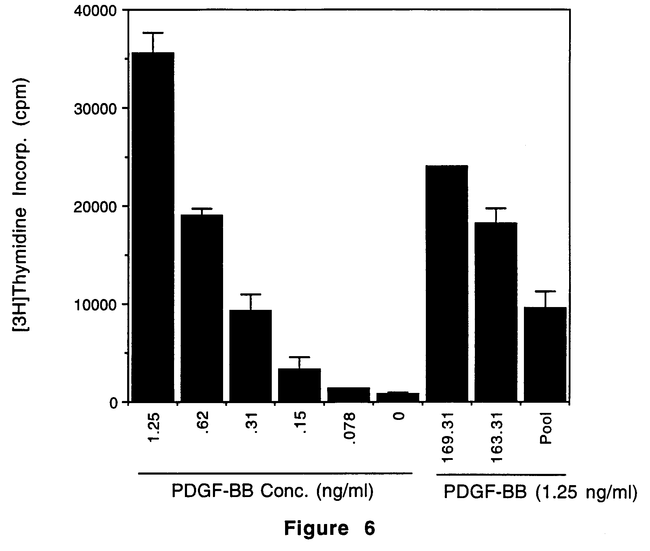

Anti-PDGF receptor MAbs were analyzed for the ability to neutralize PDGF-stimulated mitogenic activity on baboon smooth muscle cells. All mitogenic assays performed on baboon smooth muscle cells were done on primary cultures of cells between passages 3 and 7 in culture. The initial cultures were established from outgrowth of aortic tissue explants. Baboon smooth muscle cells were plated at approximately 30,000 cells per well, in DMEM supplemented with 10% fetal calf serum, into 24-well culture dishes. Two days prior to use the culture media was removed, and 1 ml of Mito Media (Table I) was added to each well to allow the cells to become quiescent. At the time of the experiment the cells were stimulated with either PDGF-AA, AB or BB. Standard curves were run for each of the three ligands using final concentrations shown in Figures 4-6. 20X stock solutions were made for each of the PDGF concentrations by dilution in 10 mM acetic acid containing 0.25% albumin, and 50 µl of PDGF or dilution vehicle alone was added to the culture wells. For the antibody neutralization studies, final PDGF concentrations of 10, 2 and 1.25 ng/ml were used for PDGF-AA, AB and BB, respectively. MAbs 163.31 and 169.31 were added to the PDGF containing wells at a final concentration of 25 µg/ml. For pools of the two antibodies, the final concentration of antibody on the cells was 25 µg/ml total, or 12.5 µg/ml for each of the MAbs. The cells were incubated between 20-24 hours at 37°C. For the PDGF-AA and AB studies, 50 µl of a 40 µCi/ml solution of [³H]thymidine was added to each well. For the PDGF-BB study the media was aspirated, then replaced with 0.5 ml of DMEM containing 5% fetal calf serum and supplemented with 2 µCi/ml of [³H]thymidine. The cells were incubated between 2-4 hours at 37°C, washed with PBS, then harvested with trypsin and counted for [³H]thymidine incorporation in a Wallac Betaplate® beta counter. As shown in Figure 4, PDGF-AA mitogenic activity was 100% neutralized by MAb 169.31 as well as by the antibody pool, but not by MAb 163.31. PDGF-AB mitogenic activity was completely inhibited by both MAbs individually as well as by the antibody pool (Figure 5). It is interesting to note that the level of [³H]thymidine incorporation in the presence of the MAbs was below the level obtained with the addition of vehicle control only. This was similarly seen with MAb 169.31 on the PDGF-AA plate (Figure 4). For the PDGF-BB stimulated cells, MAb 169.31 and MAb 163.31 gave less than 50% neutralization individually, while a pool of the two antibodies was able to neutralize approximately 75 % of the mitogenic activity (Figure 6).

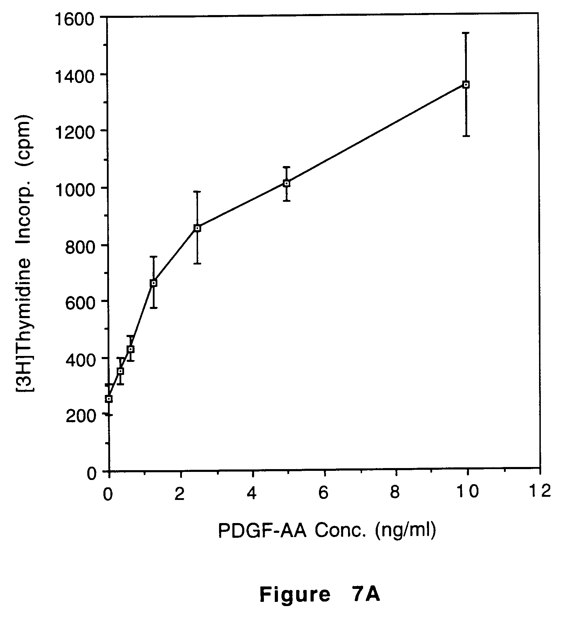

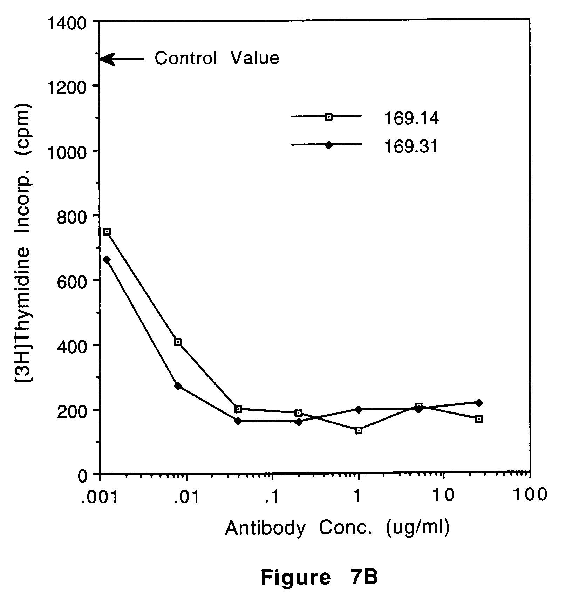

To further demonstrate the inhibitory potency of these antibodies to neutralize the mitogenic response of PDGF on baboon smooth muscle cells, two anti-PDGF-alpha receptor MAbs, 169.14 and 169.31, were analyzed for the ability to inhibit PDGF-AA mitogenic activity. Baboon smooth muscle cells were plated and treated essentially as described above. To one set of wells were added increasing concentrations of PDGF-AA in order to generate a standard curve of PDGF-AA mitogenic activity (Figure 7A). The PDGF-AA samples ranged from 10 ng/ml down to 0.31 ng/ml. To a second set of wells, a standard dilution of PDGF-AA was added to give a final concentration of 10 ng/ml. Decreasing concentrations of MAbs 169.14 and 169.31 were then added to the wells to monitor the neutralizing potency for each of the MAbs, as determined by a decrease in the level of [³H]thymidine incorporation (Figure 7B). The findings demonstrate that even at 8 ng/ml of antibody, there was greater than 90% inhibition of a 10 ng/ml solution of PDGF-AA.

### Example 9

### Neutralization of Baboon Serum Mitogenic Activity on Baboon Smooth Muscle Cells

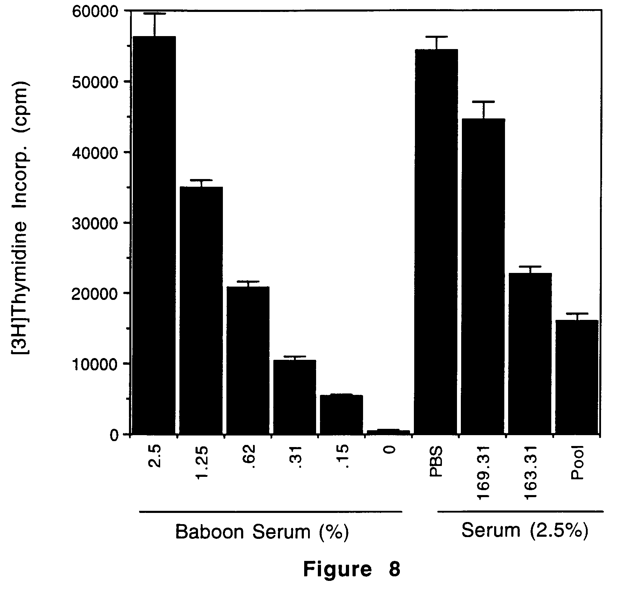

Anti-PDGF receptor MAbs were analyzed for the ability to neutralize the mitogenic potency of baboon serum on baboon smooth muscle cells. Baboon smooth muscle cells were plated at approximately 30,000 cells per well, in DMEM supplemented with 10% fetal calf serum, into 24-well culture dishes. Three days prior to use the culture media was removed, and 1 ml of Mito Media (Table I) was added to each well to allow the cells to become quiescent. At the time of the experiment the cells were stimulated with varying amounts of baboon serum. A standard curve was generated for the serum sample. 20X stock solutions were made for each of the serum concentrations, and 50 µl of the serum dilution or dilution vehicle, PBS, was added to the culture wells to give final serum concentrations on the cells ranging from 2.5% down to 0.15%. MAbs 169.31 and 163.31 were analyzed for the ability to neutralize baboon serum mitogenic activity. A final serum concentration of 2.5% was used for the antibody neutralization studies. MAbs 169.31 and 163.31 were added to the serum-containing wells at a final concentration of 25 µg/ml. For pools of the two antibodies, the final concentration of antibody on the cells was 25 µg/ml total, or 12.5 µg/ml for each of the MAbs. The cells were incubated with the serum samples for approximately 20 hours at 37°C. At that time the media was aspirated from the cells, then replaced with 0.5 ml of DMEM containing 5% fetal calf serum and supplemented with 2 µCi/ml of [³H]thymidine. The cells were incubated for approximately 3 hours at 37°C, washed with PBS, then harvested with trypsin and counted for [³H]thymidine incorporation in a Wallac Betaplate® beta counter. The results, presented in Figure 8, demonstrate that baboon serum mitogenic activity is minimally neutralized by MAb 169.31, but neutralized greater than 50% by MAb 163.31. The pool of the two antibodies inhibited greater than 75% of the serum mitogenic activity.

These results demonstrate that the majority of the mitogenic activity in baboon serum towards baboon smooth muscle cells can be inhibited through the use of anti-PDGF receptor monoclonal antibodies. Studies by the inventors have shown that the predominant form of PDGF in baboon platelets is PDGF-BB. Due to the large percentage of PDGF-beta receptors on baboon smooth muscle cells, it is consistent that the anti-PDGF-beta receptor MAb would have the largest inhibitory activity towards baboon serum.

### Example 10

### The Effect of Circulating MAb 169.31 on Baboon Serum Mitogenic Activity

A study was performed to monitor the circulating levels of MAb 169.31 after the administration of a bolus injection of 25 mg i.v. into a baboon. Serum was obtained at various intervals following antibody injection, and the level of circulating antibody was determined by ELISA. Sheep anti-mouse IgG was added to 96-well microtiter dishes in ELISA buffer A at a concentration of 2 µg/ml. The plates were incubated overnight at 4°C, washed with ELISA C buffer, then incubated with ELISA B buffer to block nonspecific binding sites. The plates were washed with ELISA C buffer, then incubated with 100 µl/well of test sample. Baboon plasma or serum containing monoclonal antibody 169.31 was diluted 1:1000 with ELISA B buffer and added to the test wells. Standards, consisting of purified MAb 169.31 spiked into control baboon plasma or serum, were diluted 1:1000, similar to the test plasma/serum samples, then added to the test wells. Standards ranged from 100 ng/ml to 1.56 ng/ml final concentration in the test wells. The plasma/serum samples were incubated in the wells for 1-2 hours at 37°C, the wells washed with ELISA C buffer, then goat anti-mouse IgG conjugated with horseradish peroxidase was added. The wells were incubated for 1 hour at 37°C, washed with ELISA C buffer, then incubated with Reaction buffer. The reaction was stopped by the addition of 1 N H₂SO₄, and the plates were read in an ELISA plate reader at 490 nm.

In addition, the relative mitogenic potency of the serum samples obtained at 1 hour and 18 hours following the injection was determined. At this time the circulating levels of antibody in the baboon were determined to be 46 µg/ml and 21 µg/ml, respectively. The 1 hour and 18 hour serum samples were then compared to a control serum sample for relative mitogenic activity. Dilutions of the serum samples were added to baboon smooth muscle cells that had been cultured essentially as described in the baboon serum study presented above. The final serum concentrations on the smooth muscle cells ranged from 1.25% down to 0.15%. The baboon smooth muscle cells were monitored for the level of [³H] thymidine incorporation, as a means to determine mitogenic activity, essentially as described above.

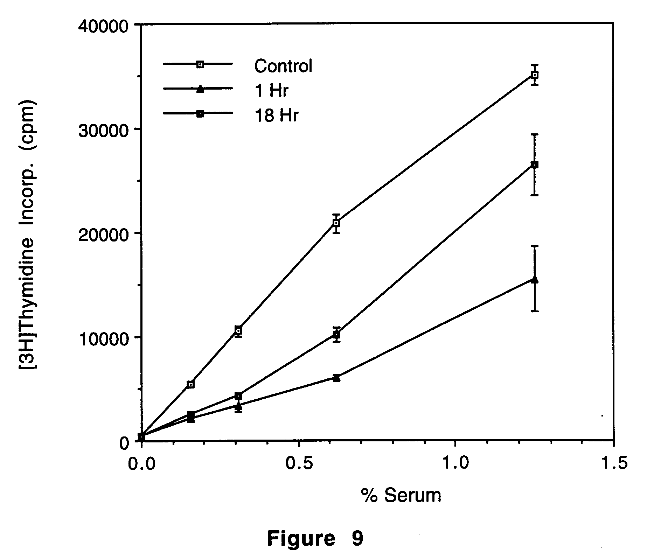

The results, presented in Figure 9, show that there was a significant decrease in the relative mitogenic potency for both the 1 hour and 18 hour serum samples as compared to the control sample, with the 18 hour sample being intermediate in mitogenic activity between the control and 1 hour samples. The level of neutralization by MAb 169.31 that was present in the 18 hour serum sample is consistent with the level of neutralization obtained when this antibody was added ex vivo to control baboon serum (Figure 8). Thus, these results demonstrate that MAb 169.31 circulating for at least 18 hours in baboon blood retains essentially all of its biological activity for neutralizing baboon serum mitogenic activity on baboon smooth muscle cells.

### Example 11

### Neutralization of Cell Outgrowth from Baboon Aortic Explants

Anti-PDGF receptor monoclonal antibodies were tested for the ability to decrease the rate of smooth muscle cell outmigration from explants of baboon aortic tissue. The inner media of the thoracic aorta of baboons was dissected out in DMEM culture media containing 10 mM Hepes. The aortic tissue was sectioned into 1 mm square sections, and the explants were placed onto tissue culture flasks. After a 10 minute incubation to allow time for the explants to adhere to the flasks, culture media, DMEM plus 6 µg/ml insulin, 5 µg/ml transferin and 10 mM Hepes, was added to the explants, and the samples were incubated at 37°C with 5% C0₂. A total of 15 explants were set up in each culture flask. At various times following the establishment of the explants the explants were examined under a high power microscope to count the number of explants that had visible cell outgrowth onto the culture dish. Explants were counted as positive if at least one cell migrated from the explant tissue out onto the culture dish surface. Explants were followed for at least seven days. In experiment #1, the explants were cultured in the DMEM culture media supplemented with insulin and transferrin containing the following test samples: 1) Anti-PDGF alpha receptor MAb (169.31) at 50 µg/ml; 2) Anti-PDGF beta receptor MAb (163.31) at 50 µg/ml; or 3) DMEM media alone (control). In experiment #2 the extracts were cultured in DMEM plus insulin and transferrin, and either 1) Pool of anti-PDGF alpha and beta receptor MAbs (169.31 and 163.31) at 25 µg/ml each; or 2) DMEM media alone (control)

The results, presented as the mean percentage of explants positive for cell outgrowth +/- SEM for each test condition (Table V), demonstrate that the anti-PDGF receptor monoclonal antibodies, individually as well as in pools, are able to decrease the level of smooth muscle cell outgrowth from the baboon aortic explants when measured at both four and seven days. These finding indicate that these antibodies are able to inhibit processes required for cell migration through a solid matrix, such as could be required for cells to migrate through existing vascular tissue toward sites of intimal hyperplasia.

**Table V**

| Migration of Vascular Smooth Muscle Cells Out of Baboon Aortic Tissue Explants Experiment #1 | | | |
|---|---|---|---|
| Sample | (n) | Day 4 | Day 7 |
| Control | 7 | 11 +/- 4 | 61 +/- 4 |
| MAb 169.31 | 7 | 6 +/- 2 | 43 +/- 5 |
| MAb 163.31 | 7 | 6 +/- 5 | 38 +/- 5 |

| Experiment #2 | | | |
|---|---|---|---|
| Sample | (n) | Day 4 | Day 7 |
| Control | 11 | 9 +/-3 | 58 +/- 4 |
| Pool | 11 | 2 +/- 1 | 36 +/- 6 |
| (n): number of times experiment repeated. | | | |

### Example 12

### Neutralization of PDGF Mitogenic Activity on Human Dermal Fibroblasts by Delayed Addition of Anti-PDGF Receptor Monoclonal Antibodies

Human dermal fibroblasts were plated at approximately 20,000 cells per well in 24-well culture dishes and grown until quiescent in DMEM containing 2% fetal calf serum. The cells were stimulated with either PDGF-AA, AB or BB. Increasing concentration of each of the PDGF ligands were added to the cells to generate standard curves of mitogenic potency for the three PDGF isoforms. The final PDGF concentrations used for the standards were 5, 2.5, 1.25, 0.62, 0.31, 0.15 and 0.0 ng/ml. 50x stock solutions of PDGF were made in 10 mM acetic acid containing 0.25% rabbit serum albumin. 25 µl of each of the stock solutions were added to triplicate test wells. To look for neutralizing acitivy by MAbs 162.62 and 169.14, wells containing the fibroblasts were incubated with 5 ng/ml of PDGF, final concentration. At various time intervals following the addition of the PDGF samples (1, 2, 4, 6 and 8 hours), a pooled sample of MAb 162.62 and MAb 169.14, 25 µg/ml final concentration for each MAb, was added to triplicate wells of the cells that had been treated with 5 ng/ml of PDGF. Nine hours after the addition of the PDGF samples, 50 µl of [³H]thymidine, 20 µCi/ml in DMEM containing 1% fetal calf serum, was added to each well. The samples were incubated for an additional 13-15 hours at 37°C. The cells washed with PBS, then harvested with trypsin and counted in a Beta plate counter.

The results, presented in Table VI, are given as mean cpm of [³H]thymidine incorporated, +/- standard deviation, for triplicate determinations. The data are given for both the PDGF standard curves, and for the time course of antibody addition. The results demonstrate that there was a 75% decrease in the mitogenic activity for PDGF-AA when the anti-PDGF receptor antibodies were added to the cells as late as 8 hours following the addition of PDGF ligand. For both PDGF-AB and BB, the addition of the anti-PDGF receptor antibodies 8 hours after the addition of PDGF ligand caused a greater than 90% decrease in PDGF mitogenic activity. These studies demonstrated that the anti-PDGF receptor monoclonal antibodies can be added to cells at prolonged times after the presence of PDGF ligand and still have potent neutralizing effects against PDGF mitogenic activity.

**Table VI**

| PDGF-AA (ng/ml) | | | MAbs 162.62/169.14 (PDGF-AA, 5 ng/ml) | | |
|---|---|---|---|---|---|
| ng/ml | cpm +/- | (st.dev.) | Time in Hrs | cpm +/- | (st.dev.) |
| 5.0 | 7888 | (768) | 1 | 5400 | (870) |
| 2.5 | 7892 | (460) | 2 | 4350 | (431) |
| 1.25 | 6044 | (1126) | 4 | 5323 | (574) |
| 0.62 | 5569 | (315) | 6 | 5300 | (768) |
| 0.31 | 5072 | (224) | 8 | 6028 | (276) |
| 0.15 | 4888 | (393) | | | |
| 0.0 | 4804 | (320) | | | |

| PDGF-AB (ng/ml) | | | MAbs 162.62/169.14 (PDGF-AB, 5ng/ml) | | |
|---|---|---|---|---|---|
| ng/ml | cpm +/- | (st.dev) | Time in Hrs | cpm +/- | (st. dev.) |
| 5.0 | 16370 | (409) | 1 | 4372 | (443) |
| 2.5 | 16621 | (878) | 2 | 4783 | (401) |
| 1.25 | 14061 | (1066) | 4 | 4363 | (427) |
| 0.62 | 11238 | (238) | 6 | 5238 | (611) |
| 0.31 | 9206 | (428) | 8 | 5659 | (667) |
| 0.15 | 8061 | (1054) | | | |
| 0.00 | 5253 | (443) | | | |

| PDGF-BB (ng/ml) | | | MAbs 162.62/169.14 (PDGF-BB, 5ng/ml) | | |
|---|---|---|---|---|---|
| ng/ml | cpm +/- | (st.dev) | Time in Hrs | cpm +/- | (st. dev.) |
| 5.0 | 12427 | (1366) | 1 | 2811 | (291) |
| 2.5 | 15445 | (977) | 2 | 3076 | (169) |
| 1.25 | 13712 | (976) | 4 | 4298 | (574) |
| 0.62 | 11989 | (1248) | 6 | 5089 | (420) |
| 0.31 | 9482 | (2089) | 8 | 7335 | (502) |
| 0.15 | 6905 | (456) | | | |
| 0.00 | 3090 | (272) | | | |

### Example 13

### Displacement of Receptor-bound¹²⁵I-PDGF from Human Osteosarcoma Cells by Anti-PDGF Receptor MAbs

The four MAbs 162.62, 163.31, 169.14 and 169.31 were analyzed for the ability to displace ¹²⁵I-PDGF-AA and ¹²⁵I-PDGF-BB bound to PDGF receptors on monolayers of human osteosarcoma cells (ATCC CRL1427). Monolayers of human osteosarcoma cells, grown in 24-well culture plates, were incubated for 1 hour at 4°C with ¹²⁵I-PDGF-AA or ¹²⁵I-PDGF-BB diluted in binding media. The cells were washed with PBS, then 1 ml of either binding media alone, MAb 169.14, 169.31, 162.62, 163.31, or a pool of 169.31 and 162.62 was added to each well. The antibodies were diluted in binding media and added to the cells at a concentration of 5 µg/ml, 1 ml/well. The cells were washed for 1 hour at 4°C, then with PBS, incubated with extraction buffer, then harvested and counted in a gamma counter to monitor the level of ¹²⁵I-PDGF binding. 100 ng/ml of PDGF-BB was added to triplicate wells with ¹²⁵I-PDGF-AA and ¹²⁵I-PDGF-BB to determine the levels of non-specific binding. The results, presented in Table VII, are shown as specific cpm bound (std. dev.) for 125I-PDGF-AA and 125I-PDGF-BB following the second incubation with the test compounds listed. The % displacement value was determined by comparing the cpm bound for the test samples compared to the cpm bound in the binding medium alone wells. The results demonstrate that the anti-PDGF alpha receptor MAbs, 169.14 and 169.31, were able to displace approximately 63% of the prebound ¹²⁵I-PDGF-AA. In contrast, the anti-PDGF beta receptor MAbs, 162.62 and 163.31, had essentially no effect, displacing less than 10% of the counts. For ¹²⁵I-PDGF-BB binding, MAbs 169.14 and 169.31 were able to displace between 22-25% of the prebound counts while MAb 162.62 was able to displace 34% of the counts. The pool of 169.31 and 162.62 displaced 44% of the prebound ¹²⁵I-PDGF-BB. These results show that, in addition to being able to block PDGF binding, the anti-PDGF receptor MAbs are also able to displace prebound PDGF-AA and BB from cell-surface receptors.

**Table VII**

| Ability of anti-PDGF Receptor MAbs to Displace Recptor-bound ¹²⁵I-PDGF-AA and ¹²⁵I-PDGF-BB from Human Osteocarcinoma Cells | | | |
|---|---|---|---|
| First Inc. | Second Inc. | CPM Bound | % Displacement |
| ¹²⁵I-PDGF-AA | Binding Media | 458 (46) | 0 |
| " | 169.14 | 164 (91) | 64 |
| " | 169.31 | 174 (58) | 62 |
| " | 162.62 | 415 (18) | 9 |
| " | 163.31 | 420 (40) | 8 |
| " | 169.31/162.62 | 116 (24) | 75 |
| ¹²⁵I-PDGF-BB | Binding Media | 528 (41) | 0 |
| " | 169.14 | 411 (87) | 22 |
| " | 169.31 | 395 (30) | 25 |
| " | 162.62 | 349 (48) | 34 |
| " | 163.31 | 518 (129) | 2 |
| " | 169.31/162.62 | 289 (58) | 44 |

Although the foregoing invention has been described in some detail by way of illustration and example for purposes of clarity of understanding, it will be evident that certain changes and modifications may be practiced within the scope of the appended claims.

## Claims

1. Use of an anti-PDGF receptor antibody which inhibits binding of PDGF to the PDGF receptor for the preparation of a medicament for use in inhibiting intimal hyperplasia in the vasculature of a mammal, subject to the limitation that said antibody does not specifically bind to the fifth Ig-like domain of the human PDGF-beta receptor.

2. Use according to claim 1 wherein said mammal is a primate.

3. Use according to claim 1 wherein said antibody is a monoclonal antibody.

4. Use according to claim 1 wherein said antibody is an anti-PDGF-alpha receptor antibody.

5. Use according to claim 1 wherein said antibody is an anti-PDGF-beta receptor antibody.

6. Use according to claim 1 of an anti-PDGF receptor antibody which inhibits binding of PDGF to the PDGF receptor for the preparation of a medicament for administration to a mammal prior to an acute vascular injury, for inhibiting intimal hyperplasia in the vasculature of the mammal, subject to the limitation that said antibody does not specifically bind to the fifth Ig-like domain of the human PDGF-beta receptor.

7. Use according to claim 6 wherein said injury is due to vascular reconstruction.

8. Use according to claim 7 wherein said vascular reconstruction comprises angioplasty, endarterectomy, atherectomy, or vascular graft emplacement.

9. Use according to any of claims 1 to 5 wherein said antibody is administered prior to or in combination with emplacement of a vascular graft.

10. Use according to any of claims 1 to 5 wherein said antibody is administered subsequent to an acute vascular injury.

11. Use according to claim 10 wherein said injury is due to vascular reconstruction.

12. Use according to claim 11 wherein said vascular reconstruction comprises angioplasty, endarterectormy, atherectomy or vascular graft emplacement.

13. Use according to claim 1 of a panel of anti-PDGF receptor antibodies which inhibit binding of PDGF to the PDGF receptor for the preparation of a medicament for use in inhibiting intimal hyperplasia in the vasculature of a mammal, wherein said panel of anti-PDGF receptor antibodies is capable of neutralizing the AA, AB and BB isoforms of PDGF to PDGF receptors, subject to the limitation that said panel of antibodies does not specifically bind to the fifth Ig-like domain of the human PDGF-beta receptor.

14. Use according to claim 13 wherein said panel of anti-PDGF receptor antibodies comprises anti-PDGF-alpha receptor antibodies and anti-PDGF-beta receptor antibodies.

15. Use according to any of claims 1 to 11 wherein said antibody is a humanized monoclonal antibody.

16. Use according to any of claims 1 to 11 wherein said antibody is a single chain antibody.

17. Use according to any of claims 1 to 11 wherein said antibody is a chimeric antibody.

18. Use according to claim 17 wherein said antibody is a human-mouse chimeric antibody.

19. Use according to claim 18 wherein said chimeric antibody comprises mouse variable domains operably linked to human constant domains.

20. Use according to any of claims 1 to 6 or 13 to 14 wherein said intimal hyperplasia occurs within a vascular graft or transplanted organ.

21. Use according to claim 1 of an anti-PDGF receptor antibody which inhibits binding of PDGF to the PDGF receptor for the preparation of a medicament for use in inhibiting smooth muscle cell proliferation at a site of vascular injury in a mammal, wherein said antibody is selected from the group consisting of anti-PDGF-alpha receptor antibodies and anti-PDGF-beta receptor antibodies, subject to the limitation that said antibody does not specifically bind to the fifth Ig-like domain of the human PDGF-beta receptor.

22. Use according to claim 21 wherein said mammal is a primate.

## Patentansprüche

1. Verwendung eines anti-PDGF-Rezeptor-Antikörpers, der die Bindung von PDGF an den PDGF-Rezeptor hemmt, zur Herstellung eines Arzneimittels zur Verwendung bei der Hemmung der Intima-Hyperplasie im Gefäßsystem eines Säugetiers, mit der Einschränkung, daß der Antikörper nicht spezifisch an die fünfte Ig-artige Domäne des humanen PDGF-beta-Rezeptors bindet.

2. Verwendung nach Anspruch 1, wobei es sich bei dem Säugetier um einen Primaten handelt.

3. Verwendung nach Anspruch 1, wobei es sich bei dem Antikörper um einen monoklonalen Antikörper handelt.

4. Verwendung nach Anspruch 1, wobei es sich bei dem Antikörper um einen anti-PDGF-alpha-Rezeptor-Antikörper handelt.

5. Verwendung nach Anspruch 1, wobei es sich bei dem Antikörper um einen anti-PDGF-beta-Rezeptor-Antikörper handelt.

6. Verwendung eines anti-PDGF-Rezeptor-Antikörpers nach Anspruch 1, der die Bindung von PDGF an den PDGF-Rezeptor hemmt, zur Herstellung eines Arzneimittels zur Verabreichung an ein Säugetier vor einer akuten Gefäßschädigung zur Hemmung einer Intima-Hyperplasie im Gefäßsystem des Säugetiers, mit der Einschränkung, daß der Antikörper nicht spezifisch an die fünfte Ig-artige Domäne des humanen PDGF-beta-Rezeptors bindet.

7. Verwendung nach Anspruch 6, wobei die Schädigung auf eine Gefäßrekonstruktion zurückzuführen ist.

8. Verwendung nach Anspruch 7, wobei die Gefäßrekonstruktion eine angioplastische Maßnahme, eine Endarteriektomie, eine Atheroektomie oder das Einsetzen eines Gefäßtransplantats umfaßt.

9. Verwendung nach einem der Ansprüche 1 bis 5, wobei der Antikörper vor oder in Kombination mit dem Einsetzen eines Gefäßtransplantats verabreicht wird.

10. Verwendung nach einem der Ansprüche 1 bis 5, wobei der Antikörper im Anschluß an eine akute Gefäßschädigung verabreicht wird.

11. Verwendung nach Anspruch 10, wobei die Schädigung auf eine Gefäßrekonstruktion zurückzuführen ist.

12. Verwendung nach Anspruch 11, wobei die Gefäßrekonstruktion eine angioplastische Maßnahme, eine Endarteriektomie, eine Atheroektomie oder das Einsetzen eines Gefäßtransplantats umfaßt.

13. Verwendung nach Anspruch 1 einer Gruppe von anti-PDGF-Rezeptor-Antikörpern, die die Bindung von PDGF an den PDGF-Rezeptor hemmen, zur Herstellung eines Arzneimittels zur Verwendung bei der Hemmung einer Intima-Hyperplasie im Gefäßsystem eines Säugetiers, wobei die Gruppe von anti-PDGF-Rezeptor-Antikörpern zur Neutralisation der AA-, AB- und BB-Isoformen von PDGF an PDGF-Rezeptoren befähigt ist, mit der Einschränkung, daß die Antikörper oder die Gruppe von Antikörpern nicht spezifisch an die fünfte Ig-artige Domäne des humanen PDGF-beta-Rezeptors bindet.

14. Verwendung nach Anspruch 13, wobei die Gruppe von anti-PDGF-Rezeptor-Antikörpern anti-PDGF-alpha-Rezeptor-Antikörper und anti-PDGF-beta-Rezeptor-Antikörper umfaßt.

15. Verwendung nach einem der Ansprüche 1 bis 11, wobei es sich beim Antikörper um einen humanisierten monoklonalen Antikörper handelt.

16. Verwendung nach einem der Ansprüche 1 bis 11, wobei es sich beim Antikörper um einen einzelkettigen Antikörper handelt.

17. Verwendung nach einem der Ansprüche 1 bis 11, wobei es sich beim Antikörper um einen chimären Antikörper handelt.

18. Verwendung nach Anspruch 17, wobei es sich beim Antikörper um einen chimären Human-Maus-Antikörper handelt.

19. Verwendung nach Anspruch 18, wobei der chimäre Antikörper variable Mäuse-Domänen umfaßt, die funktionell mit humanen konstanten Domänen verknüpft sind.

20. Verwendung nach einem der Ansprüche 1 bis 6 oder 13 bis 14, wobei die Intima-Hyperplasie innerhalb eines Gefäßtransplantats oder eines transplantierten Organs erfolgt.

21. Verwendung nach Anspruch 1 eines anti-PDGF-Rezeptor-Antikörpers, der die Bindung von PDGF an den PDGF-Rezeptor hemmt, zur Herstellung eines Arzneimittels zur Verwendung bei der Hemmung der Proliferation von glatten Muskelzellen an der Stelle der Gefäßschädigung bei einem Säugetier, wobei der Antikörper aus der Gruppe anti-PDGF-alpha-Rezeptor-Antikörper und anti-PDGF-beta-Rezeptor-Antikörper ausgewählt ist, mit der Einschränkung, daß der Antikörper nicht spezifisch an die fünfte Ig-artige Domäne des humanen PDGF-beta-Rezeptors bindet.

22. Verwendung nach Anspruch 21, wobei es sich beim Säugetier um einen Primaten handelt.

## Revendications

1. Utilisation d'un anticorps anti-récepteur du PDGF qui inhibe une liaison du PDGF au récepteur du PDGF pour préparer un médicament destiné à être utilisé pour inhiber l'hyperplasie de l'intima dans le système vasculaire d'un mammifère, pourvu que ledit anticorps ne se lie pas de manière spécifique au cinquième domaine, analogue à une Ig, du récepteur bêta du PDGF humain.

2. Utilisation selon la revendication 1, dans laquelle ledit mammifère est un primate.

3. Utilisation selon la revendication 1, dans laquelle ledit anticorps est un anticorps monoclonal.

4. Utilisation selon la revendication 1, dans laquelle ledit anticorps est un anticorps anti-récepteur alpha du PDGF.

5. Utilisation selon la revendication 1, dans laquelle ledit anticorps est un anticorps anti-récepteur bêta du PDGF.

6. Utilisation selon la revendication 1 d'un anticorps anti-récepteur du PDGF qui inhibe une liaison du PDGF au récepteur du PDGF pour préparer un médicament destiné à être administré à un mammifère avant une lésion vasculaire aiguë, pour inhiber l'hyperplasie de l'intima dans le système vasculaire du mammifère, pourvu que ledit anticorps ne se lie pas de manière spécifique au cinquième domaine, analogue à une Ig, du récepteur bêta du PDGF humain.

7. Utilisation selon la revendication 6, dans laquelle ladite lésion est due à une reconstruction vasculaire.

8. Utilisation selon la revendication 7, dans laquelle ladite reconstruction vasculaire comporte une angioplastie, une endartériectomie, une athérectomie, ou une mise en place d'une greffe vasculaire.

9. Utilisation selon l'une quelconque des revendications 1 à 5, dans laquelle ledit anticorps est administré avant ou pendant la mise en place d'une greffe vasculaire.

10. Utilisation selon l'une quelconque des revendications 1 à 5, dans laquelle ledit anticorps est administré après une lésion vasculaire aiguë.

11. Utilisation selon la revendication 10, dans laquelle ladite lésion est due à une reconstruction vasculaire.

12. Utilisation selon la revendication 11, dans laquelle ladite reconstruction vasculaire comporte une angioplastie, une endartériectomie, une athérectomie, ou une mise en place d'une greffe vasculaire.

13. Utilisation selon la revendication 1 d'une série d'anticorps anti-récepteur du PDGF qui inhibe une liaison du PDGF au récepteur du PDGF pour préparer un médicament destiné à être utilisé pour inhiber l'hyperplasie de l'intima dans le système vasculaire d'un mammifère, ladite série d'anticorps anti-récepteur du PDGF étant capable de neutraliser les isoformes AA, AB et BB du PDGF des récepteurs du PDGF, pourvu que lesdits anticorps ou série d'anticorps ne se lient pas de manière spécifique au cinquième domaine, analogue à une Ig, du récepteur bêta du PDGF humain.

14. Utilisation selon la revendication 13, dans laquelle ladite série d'anticorps anti-récepteur du PDGF comporte des anticorps anti-récepteur alpha du PDGF et des anticorps anti-récepteur bêta du PDGF.

15. Utilisation selon l'une quelconque des revendications 1 à 11, dans laquelle ledit anticorps est un anticorps monoclonal humanisé.

16. Utilisation selon l'une quelconque des revendications 1 à 11, dans laquelle ledit anticorps est un anticorps à chaîne unique.

17. Utilisation selon l'une quelconque des revendications 1 à 11, dans laquelle ledit anticorps est un anticorps chimère.

18. Utilisation selon la revendication 17, dans laquelle ledit anticorps est un anticorps chimère humain-murin.

19. Utilisation selon la revendication 18, dans laquelle ledit anticorps chimère comporte des domaines variables murins liés de manière opérationnelle à des domaines constants humains.

20. Utilisation selon l'une quelconque des revendications 1 à 6 ou 13 à 14, dans laquelle ladite hyperplasie de l'intima survient dans une greffe vasculaire ou un organe transplanté.

21. Utilisation selon la revendication 1 d'un anticorps anti-récepteur du PDGF qui inhibe une liaison du PDGF au récepteur du PDGF pour préparer un médicament destiné à être utilisé pour inhiber la prolifération de cellules de muscle lisse au niveau d'un site d'une liaison vasculaire chez un mammifère, ledit anticorps étant sélectionné parmi le groupe constitué des anticorps anti-récepteur alpha du PDGF et des anticorps anti-récepteur bêta du PDGF, pourvu que ledit anticorps ne se lie pas de manière spécifique au cinquième domaine, analogue à une Ig, du récepteur bêta du PDGF humain.

22. Utilisation selon la revendication 21, dans laquelle ledit mammifère est un primate.
